# NOUVEAU FASCICULE DE BREVET EUROPEEN

(11) **EP 1 312 385 B2**
(45) Date de publication et mention de la décision concernant l'opposition: **21.10.2009**
(45) Mention de la délivrance du brevet: 01.02.2006
(21) Numéro de dépôt: 02292870.9
(22) Date de dépôt: 19.11.2002
(51) Int. Cl.: A61L 29/08, A61M 25/00

(54) **Dispositif medical intraluminal**
Intraluminales medizinisches Gerät
Intraluminal medical device

(30) Priorité: 20.11.2001 FR 0114988
(43) Date de publication de la demande: 21.05.2003
(73) Titulaire: B. Braun Medical SAS, 92100 Boulogne Billancourt (FR)
(72) Inventeur: M.Collin, Rémi, 28402 Nogent-Le-Rotrou (FR); M.Bezou, Pascal, 28402 Nogent-Le-Rotrou (FR)
(74) Mandataire: Lebrette, Camille

(56) Documents cités:
- WO-A-00/67816
- WO-A-00/71181
- WO-A-96/30277
- WO-A-98/11932
- WO-A-98/19729
- GB-A- 2 319 507
- US-A- 5 001 009
- US-A- 5 509 899
- US-A- 5 800 412
- US-A- 5 800 412
- US-A- 5 895 374

## Description

L'invention concerne le domaine des dispositifs médicaux, et plus particulièrement des cathéters, ou sondes médicales, adapté(e)s pour être introduit(e)s dans un conduit corporel. Les conduits intraluminaux où circule un fluide (vaisseau sanguin, canal urinaire...) sont en particulier concernés. Les sondes urinaires ou annales sont spécialement visées.

Dans EP-A-0 935 478, est ainsi décrite une telle sonde présentant au moins localement une zone hydrophile comprenant une substance hydrophile et présentant extérieurement un faible coefficient de frottement consécutivement à un traitement de ladite zone avec un agent de mouillage de ladite substance hydrophile, préalablement à l'utilisation de la sonde.

Dans EP-A-0 935 478, l'importance d'une lutte contre une évaporation excessive de l'agent de mouillage, dans le temps, après que la sonde urinaire a été traitée avec cet agent de mouillage, est évoquée, et une solution est apportée consistant en particulier à utiliser un emballage à parois imperméables au gaz.

La présente invention traite également du problème du contrôle de l'évaporation de l'agent de mouillage une fois le dispositif médical traité, et/ ou du problème d'écoulement intempestif des emballages (voire desdits dispositifs eux-mêmes lorsqu'ils sont mouillés par l'agent de mouillage).

La solution proposée par l'invention consiste en un ensemble selon la revendication 1 et en un procédé selon la revendication 10, et en particulier en ce que la zone hydrophile comprend, à sec, une deuxième substance hydrophile constituant un agent de rétention de l'agent de mouillage au sein de la zone hydrophile, pour limiter l'évaporation à l'air de cet agent de mouillage hors du dispositif médical et/ou éviter audit agent de mouillage de couler intempestivement (comme un liquide ; de l'eau, en particulier), une fois la zone traitée.

Dans la présente description, le terme "zone" doit être compris comme tout ou partie du dispositif médical devant être introduit dans le conduit corporel concerné et en particulier comme tout ou partie de l'épaisseur de ce dispositif.

On notera également que dans la solution précitée, et plus généralement dans l'invention, il ne s'agit pas comme dans WO 00/67816 de pourvoir le dispositif médical concerné d'un agent contre l'humidification prématurée de la couche hydrophile (antiblock agent) qui serait d'abord dissout dans un solvant (par exemple de l'éthanol, de la glycérine ou de l'eau) avant d'en enduire la zone hydrophile pour constituer un revêtement de surface de cette zone une fois sec, après évaporation du solvant, l'agent de prévention (antiblock agent) formant en fin de compte un revêtement extérieur protecteur, par-dessus la couche hydrophile lubrifiante. La prévision d'un tel agent de prévention est ici inapproprié.

Dans l'invention, il ne s'agit pas non plus de retenir la solution de WO 00/71181 où l'on associe à une première couche hydrophile une deuxième couche extérieure hydrophobe comprenant un surfactant hydrophile. Notamment une telle couche hydrophobe s'avère inappropriée dans le cadre de l'invention.

Concernant la solution de l'invention telle que globalement présentée ci-avant, la deuxième substance hydrophile sera choisie comme totalement compatible (chimiquement en particulier) avec la première substance hydrophile pour constituer ce que l'invention définit comme l'agent de rétention de l'agent de mouillage qui l'imprègne, une fois la zone hydrophile traitée par contact avec cet agent de mouillage.

Par ailleurs, la seconde substance hydrophile demeure au contact du dispositif médical où elle a été appliquée, au sein de la zone hydrophile, en étant "liée" fonctionnellement en permanence à la première substance hydrophile pendant toute la durée d'utilisation du dispositif médical dans le conduit où ce dispositif a été préalablement introduit, et même d'ailleurs dans l'emballage où ce dispositif a été préalablement conditionné, dès lors que le dispositif est recouvert de ses deux substances hydrophiles, sèches, dès sa fabrication, et est donc ainsi conditionné.

Pour des raisons de technique de fabrication du dispositif médical, de fiabilité dans le temps et d'efficacité en termes de mouillage et de rétention, on conseille par ailleurs que la zone hydrophile comprenne :
- une première couche comprenant une proportion nettement plus importante de première substance hydrophile que de deuxième substance hydrophile, et
- une deuxième couche, autour de la première, comprenant une proportion nettement plus importante de deuxième substance hydrophile que de première substance hydrophile.

Dans ce contexte, l'invention traite également du problème de la nature de l'agent de rétention de l'agent de mouillage permettant d'éviter autant que possible la présence de liquide ou d'un bain de mouillage dans l'emballage, afin notamment d'éviter tout écoulement inopiné lors du déconditionnement du produit, lorsque l'emballage est ouvert pour utilisation du dispositif.

A cet égard, l'invention conseille que l'agent de mouillage ait la consistance d'un gel, de sorte que le traitement de la zone hydrophile du dispositif médical par l'agent de mouillage puisse consister en une enduction de cette zone (trempage, répartition sous vide, etc ...) par un composé "solide" ou "semi solide", en particulier visqueux, beaucoup plus visqueux qu'un liquide. Un gel de mouillage (ayant une capacité de rétention d'un composé aqueux) pourra ainsi recouvrir extérieurement la zone hydrophile du dispositif médical.

En relation avec ce qui précède, une autre caractéristique de l'invention consiste d'ailleurs en ce que le dispositif médical (dont la zone hydrophile est alors imprégnée par l'agent de mouillage), demeure dans son emballage, après ce traitement et pendant sa durée ultérieure de conditionnement dans cet emballage, sans y tremper dans aucun liquide.

A cet égard, on doit comprendre qu'il peut malgré tout y avoir des traces d'humidité dans l'emballage, ces traces étant dues au contact entre la paroi (intérieure) de l'emballage et le dispositif médical mouillé (traces du gel de mouillage en particulier).

Un autre problème que traite l'invention concerne les risques auxquels le patient pourrait être exposé, dans l'hypothèse où malgré toutes les précautions prises, le frottement entre le dispositif médical traité et le conduit corporel de ce patient s'avérerait malgré tout trop important.

Dans cette hypothèse, une caractéristique de l'invention conseille que l'agent de rétention de l'agent de mouillage comprenne dans sa composition un lubrifiant qui limite les frottements vis-à-vis du conduit d'implantation par rapport à ce que seraient ces frottements, dans les mêmes conditions, sans lubrifiant.

Parmi les corps susceptibles de constituer un tel lubrifiant, la glycérine est à conseiller pour ses qualités à la fois lubrifiante et de rétention des solutions aqueuses, ainsi que pour la possibilité offerte d'un procédé de mise en oeuvre peu onéreux, la glycérine étant de surcroît un produit médicalement couramment utilisé et de bonne tolérance.

Bien que des essais menés en particulier sur une sonde urinaire aient d'abord incité à utiliser une proportion de l'ordre de 50 % en poids de glycérine en mélange avec l'agent de mouillage, il s'est avéré qu'il était finalement préférable d'augmenter nettement la concentration en glycérine dans un tel mélange entre 70 % et 90 % environ (à 5 % près).

En relation avec ce qui précède, il est d'ailleurs plus généralement conseillé que la zone hydrophile, lubrifiante ou non, comprenne entre environ 59,5 % et 10 % de composé aqueux (à 2 % près), en tant qu'agent de mouillage et au moins 40 % environ de deuxième substance hydrophile.

Avec un tel dosage, les performances dans le temps du dispositif médical concerné, y compris lorsqu'il est placé à l'air un certain temps, hors de son emballage, demeurent très satisfaisantes.

S'il s'agit d'une sonde, le dispositif médical pourra en particulier comprendre un corps central en un polymère hydrophobe ou hydrophile revêtu d'une première fine couche hydrophile (contenant typiquement du polyvinyle pirolidone - PVP-greffé en tant que composé actif sur quelques microns d'épaisseur), cette première couche étant recouverte d'une deuxième couche hydrophile humidifiée, d'épaisseur supérieure à la première.

L'utilisation de PVP-greffé assure un maintien efficace de la première couche hydrophile sur le corps central de la sonde et la seconde couche d'enrobage utilisée en particulier pour l'humidification de la première permet d'atteindre une lubrification limitant les désagréments pour l'utilisateur et les éventuels risques corporels associés, lorsque la sonde est mouillée, avec une tenue convenable dans le temps.

A noter par ailleurs que, par souci d'asepsie, toute sonde doit être conservée stérile dans son emballage.

Comme indiqué précédemment, EP-A-0 935 478 enseigne d'utiliser un emballage étanche au gaz (feuille d'aluminium en particulier), ce qui empêche une stérilisation au gaz (typiquement, oxyde d'éthylène) et oriente typiquement vers une stérilisation par rayonnement.

En relation avec cela, l'invention vise à limiter les coûts de l'étape de stérilisation, en tirant parti de la présence de l'agent de rétention du liquide de mouillage.

Ainsi, une autre caractéristique de l'invention conseille-t-elle, pour obtenir une sonde médicale conditionnée au mieux dans son emballage :
- de préparer la sonde pour qu'à l'intérieur de l'emballage la zone hydrophile comprenne une deuxième substance hydrophile constituant un agent de rétention de l'agent de mouillage, en vue en particulier de limiter l'évaporation à l'air de cet agent de mouillage hors de la sonde, une fois la zone traitée,
- et de stériliser dans l'emballage la sonde ainsi conditionnée, par vaporisation gazeuse à travers une paroi perméable au gaz de l'emballage.

A noter que l'évaporation du liquide de mouillage qui est potentiellement plus importante, toutes choses égales par ailleurs, à travers une telle paroi qu'à travers une paroi étanche au gaz, est compensée par l'effet de rétention obtenu par l'agent de rétention qui préserve le mouillage, c'est-à-dire l'hydratation ou l'humidification, de la zone hydrophile.

On conseille également :
- que l'étape de préparation de la sonde comprenne l'enduction d'une couche comprenant la deuxième substance hydrophile autour d'une couche sous-jacente comprenant la première substance hydrophile,
- et/ou que lors de l'étape de préparation de la sonde, on mette en contact la première substance hydrophile avec à la fois la deuxième substance hydrophile et l'agent de mouillage.

Dans ce qui précède et dans le notamment d'une sonde, on aura également remarqué que le traitement de la zone hydrophile de la sonde par l'agent de mouillage s'effectue très avantageusement préalablement au, ou lors du, conditionnement de la sonde dans son emballage, ce qui permet d'obtenir un produit prêt à l'emploi : l'utilisateur n'a pas à attendre un certain "délai de mouillage" de la sonde pour l'utiliser, contrairement à ce qui est par exemple prévu dans EP-A-0 815 037.

Dans ce qui suit, une description encore plus détaillée de l'invention est présentée, en référence aux dessins d'accompagnement qui s'appliquent à l'utilisation d'une sonde intraluminale et dans lesquels :
- la figure 1 montre schématiquement une sonde conforme à l'invention, dans son état prêt à l'emploi,
- la figure 2 montre une coupe longitudinale agrandie de la sonde à l'endroit repéré II sur la figure 1,
- la figure 3 montre la sonde dans un emballage approprié pour son stockage (qui peut être de longue durée), et
- la figure 4 montre une variante de réalisation.

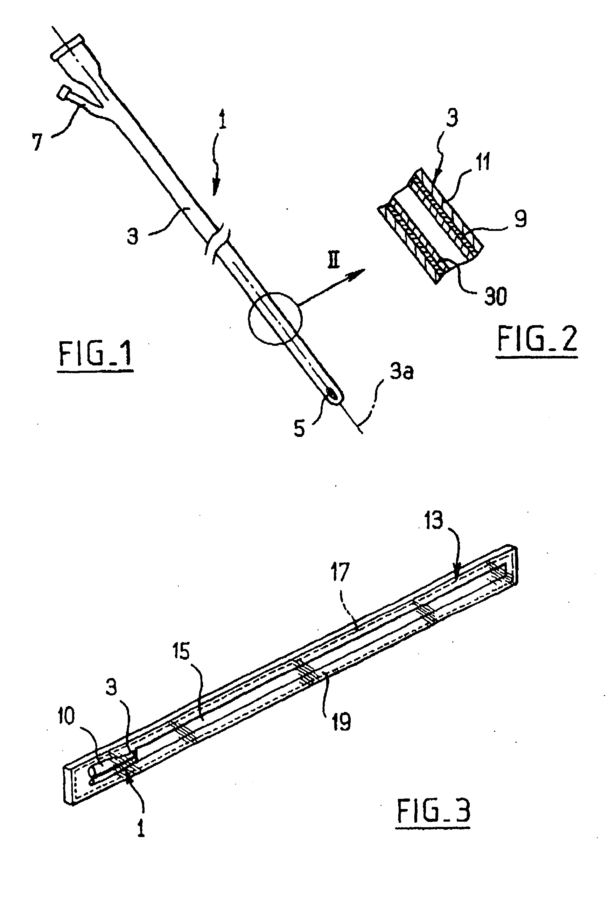

Sur la figure 1 on voit une sonde urinaire 1, en tant qu'illustration non limitative d'un dispositif médical conforme à l'invention.

Il s'agit d'une sonde traditionnelle qui se présente comme un cathéter 3 allongé suivant l'axe 3a, de flexibilité appropriée et présentant une ouverture 5 à son extrémité distale. Un bras latéral 7 de sortie d'urine se raccorde au cathéter 3, côté proximal.

La sonde urinaire, et en particulier le cathéter 3, pourrait notamment être réalisé(e) comme décrit dans CN-A-11 06 744 (voir pages 2 et 3, revendications 1 à 14). Dans ce cas, toute la structure du cathéter pourrait être constituée à sec (c'est-à-dire non traitée par l'agent de mouillage) d'un composé comprenant en particulier entre 10 % et 30 % en poids d'alcool polyvinyle (premier constituant hydrophile), de 30 % à 50 % en poids d'eau, de 5 % à 15 % en poids de sulfoxyde et de 2 % à 8 % en poids d'alkane linéaire C10 - C55, ainsi qu'entre 10 % et 30 % en poids d'un deuxième constituant hydrophile ayant plus particulièrement pour fonction de retenir l'agent de mouillage de l'alcool polyvinyle. On obtiendrait ainsi un dispositif, et plus particulièrement une sonde, suffisamment structuré(e) mécaniquement pour être utilisable dans de bonnes conditions de sécurité, tout en offrant extérieurement (et plus généralement sur toute l'épaisseur de la paroi du cathéter ainsi constitué) une structure hydrophile adaptée pour présenter une bonne capacité de rétention de l'agent de mouillage et une bonne performance de glissance (c'est-à-dire, un faible coefficient de frottement) consécutivement à la mise en contact de cette structure hydrophile avec l'agent de mouillage (par exemple de l'eau ou une solution aqueuse isotonique de chlorure de sodium et d'eau stérile) venant activer en particulier le composé d'alcool polyvinyle, ceci préalablement à l'utilisation de la sonde dans le conduit corporel du patient.

Une matière élastomère à capacité renforcée de rétention d'eau (utilisation de deux substances hydrophiles associées en synergie) pourrait également être employée.

Le résultat obtenu serait une sonde présentant un coefficient de frottement et une capacité de rétention de l'agent de mouillage plus faible qu'une sonde sèche non traitée par son agent de mouillage, et plus faible également qu'une sonde hydrophobe (au moins en surface extérieure) lubrifiée par exemple à la paraffine ou à la glycérine.

Pour tout détail complémentaire concernant la préparation d'une sonde à cathéter globalement hydrophile, y compris "à coeur", on se reportera notamment à l'exemple 1, pages 10 et 11 de CN-A-11 06 744.

Malgré tout, l'invention conseille de concevoir de préférence la sonde urinaire comme suit et comme illustrée en section agrandie sur la figure 2.

Tout d'abord, on fabrique une structure tubulaire ayant la forme de la sonde de la figure 1 et dont la partie appartenant au cathéter principal est repérée 30 sur la figure 2.

La matière utilisée peut notamment être du PVC (polychlorure de vinyle) ou de l'élastomère, ou du polyuréthane.

Autour de ce substrat est appliquée une couche de polymère ou de résine hydrophile présentant une grande compatibilité avec les tissus du corps humain et donc adaptée pour s'imprégner d'une solution de mouillage, si elle est mise en contact avec une telle solution.

Parmi les résines hydrophiles susceptibles de constituer la couche 9 (autrement dit, le premier constituant hydrophile de la sonde), on peut citer des polymères ou des copolymères ayant comme principal composant actif du PVP ou de l'HEMA (hydroxyéthylméthacrylate). D'autres composés utilisables peuvent être trouvés en colonne 4, ligne 6 à colonne 5, ligne 42 de JP-B-5512 265 (correspondant à JP-A-49-13 2888).

Une méthode possible de préparation du revêtement hydrophile 9 est présentée dans US-A-5 001 009.

Dans la littérature, il a déjà été proposé un procédé pour assurer une retenue mécanique d'une couche hydrophile sur un substrat tubulaire, afin d'éviter que lors de l'utilisation de la sonde, la couche de revêtement hydrophile ne se sépare du reste de la sonde, par exemple consécutivement à l'introduction de la sonde dans un conduit urinaire.

Ainsi, il a déjà été proposé de greffer chimiquement une couche hydrophile sur un substrat.

Typiquement, il est connu de conditionner une telle sonde dans un emballage approprié et, à un moment donné préalable à l'utilisation de la sonde dans le corps du patient, de mettre en contact le revêtement hydrophile 9 avec un agent de mouillage qui va imprégner la couche 9 et donc l'activer la rendant glissante, c'est-à-dire en diminuant le coefficient de frottement par rapport à ce qu'il était lorsque la surface hydrophile était sèche.

Typiquement, un tel agent liquide de mouillage est de l'eau ou une solution aqueuse.

Dans l'art antérieur, il a en outre déjà été proposé :
- soit de conditionner la sonde avec son revêtement hydrophile déjà mouillé par un liquide de gonflement dans son emballage, de sorte que la sonde peut être directement utilisée au moment de l'ouverture de l'emballage (voir EP-B-0 935 478),
- soit de mouiller la surface hydrophile juste avant l'utilisation de la sonde (comme par exemple dans US-A-3 967 728 ou dans EP-B-0 815 037 où une sonde à revêtement hydrophile est conditionnée sèche dans son emballage puis mouillée au moment de l'ouverture de cet emballage, de sorte qu'il faut attendre quelques instants avant de pouvoir utiliser la sonde, une fois l'emballage ouvert ; typiquement de l'ordre de 30 s).

L'invention conseille la seconde solution pour obtenir une sonde prête à l'emploi dès l'ouverture de l'emballage, avec toutefois une possibilité complémentaire consistant en ce que, dans l'emballage, la sonde peut ne tremper dans aucun liquide, et en particulier pas dans un liquide de mouillage (ou de gonflement) comme c'est le cas dans EP-B-0 935 478.

Pour cela, on conseille dans l'invention de traiter la surface hydrophile 9 (et plus préalablement le premier constituant hydrophile précité) avec un agent non liquide, en particulier très visqueux, ayant de préférence une consistance gélatineuse et comprenant un liquide de mouillage (en particulier de l'eau ou un composé aqueux) et un agent (appelé également deuxième constituant hydrophile) de rétention de ce liquide de mouillage limitant l'évaporation à l'air du liquide de mouillage hors de la sonde.

Ainsi, le liquide de mouillage du premier constituant hydrophile (encore sec) de la sonde va être retenu autour du cathéter, à son contact.

Un liquide de trempage tel que divulgué dans EP-B-0 935 478 ou dans US-A-3 967 728 (poche à liquide 17) devient inutile.

On évite ainsi notamment que du liquide se répande inopinément hors de l'emballage, lors de l'ouverture de celui-ci, ce qui améliore les conditions d'utilisation de la sonde.

Il est à noter que si, comme dans le cas d'un premier constituant hydrophile ayant comme composé actif principal du PVP ou de l'HEMA, on se trouve en présence d'une substance adaptée pour gonfler au contact de l'agent de mouillage, ce dernier consistera alors en un agent de gonflement, comme cela est le cas dans EP-B-0 935 478.

En tant qu'agent de mouillage pouvant avoir un effet d'agent de gonflement vis-à-vis d'une substance hydrophile adaptée, on peut citer une solution aqueuse, une solution aqueuse isotonique, une solution aqueuse isotonique de chlorure de sodium et d'eau stérile.

Sur la figure 2, le revêtement hydrophile 9 est au contact et recouvert par une couche extérieure 11 dont la fonction est donc de mouiller le revêtement hydrophile 9, tout en favorisant la retenue de l'agent de mouillage sur la sonde elle-même. Avec la consistance d'un gel, une telle couche 11 pourra facilement être répandue et ainsi enduire extérieurement la première couche hydrophile 9.

Dans le cas de la figure 2, la couche extérieure 11 définit la surface extérieure du cathéter et contient à la fois un liquide de mouillage du revêtement 9 (typiquement eau) et l'agent de rétention de ce liquide de mouillage.

Dans l'invention, l'agent de rétention du liquide de mouillage sera de préférence un lubrifiant ayant donc une capacité hygroscopique.

Comme tout lubrifiant, celui-ci aura pour fonction de limiter les frottements (notamment au contact d'un conduit corporel), en particulier dans l'hypothèse où la diminution du coefficient de frottement de la sonde induite par le mouillage ne serait pas jugée suffisante au moment de l'utilisation de cette sonde.

Une substance à la fois lubrifiante et de rétention de l'agent de mouillage du premier constituant hydrophile est la glycérine. Bien que moins performants, on pourrait tenter d'utiliser d'autres glycols ou tryols alphatiques à capacité lubrifiante et de masse molaire inférieure à 200.

Quoi qu'il en soit, l'agent de rétention du liquide de mouillage, de par son pouvoir hygroscopique, maintient hydratée la couche hydrophile 9, en réduisant l'évaporation de l'eau.

Contrairement à ce qui avait été d'abord imaginé, les meilleurs résultats ont été obtenus en utilisant, pour la couche 11, un composé comprenant entre 54,5 % et 10 % de liquide de mouillage (eau, en particulier) et au moins 45 % d'agent de rétention de ce liquide de mouillage (de préférence, entre 70 % et 90 % de glycérine). Dans le temps, les caractéristiques de rétention d'eau et de glissance se sont avérées les meilleures, tout en montrant une bonne résistance à la stérilisation, dans le cas d'une stérilisation gazeuse à l'oxyde d'éthylène.

L'enduction de la couche hydrophile 9 par un gel susceptible de former la couche 11 de la figure 2 peut être obtenue par trempage du revêtement 9 (et donc du cathéter 30) dans un bain de ce gel. Une autre technique assurant une répartition uniforme de l'agent mouillant autour de la couche 9, en recourant par exemple à une mise en dépression d'une gaine où a été glissée préalablement la sonde et qui contiendrait une certaine quantité dudit gel, peut également être utilisée.

A cet égard, l'épaisseur de la couche 9 peut être de quelques microns, celle de la couche gélifiée 11 pouvant être de 3 à 10 fois supérieure.

Etant donné que l'invention conseille de proposer à l'utilisateur une sonde urinaire "prête à l'emploi", l'enduction de la couche 9 par la couche extérieure 11 s'effectuera de préférence avant ou au moment du conditionnement de la sonde dans un emballage, tel que l'emballage 13 de la figure 3, de sorte que pendant toute sa durée de conservation dans l'emballage stérile 13 (durée comprise typiquement entre quelques mois et plusieurs années), la sonde 1 sera imprégnée extérieurement de l'agent de mouillage et, ainsi, il suffira d'ouvrir l'emballage 13 pour utiliser immédiatement cette sonde, sans avoir à attendre alors un certain temps d'imprégnation de la surface hydrophile.

Dans l'exemple de la figure 3, la sonde 1 est, à l'intérieur de l'emballage 13, logée dans une gaine tubulaire 15. En l'espèce, toute la longueur du cathéter 3 s'étend à l'intérieur de la gaine 15 ; seule l'extrémité proximale 10 de la sonde ressort.

A l'intérieur de la gaine 15, le cathéter 3 ne trempe donc dans aucun liquide, de même qu'aucun liquide n'est contenu dans la poche intérieure 17 de l'emballage où est placée la gaine 15.

Une fois la sonde 1 glissée dans la gaine 15 et cet ensemble placé dans la poche 17, l'emballage 13 peut être fermé.

On notera que l'une au moins des parois de l'emballage, telle que la paroi frontale 19 sur la figure 3, est de préférence perméable au gaz, et en particulier au gaz utilisé pour la stérilisation (tel que l'oxyde d'éthylène).

Ainsi, une fois l'emballage 13 scellé autour de la sonde, une telle stérilisation au gaz va pouvoir être réalisée à travers la paroi 19.

On notera qu'en dépit de cette perméabilité au gaz, la présence de l'agent de rétention du liquide de mouillage au contact dudit premier constituant hydrophile de la sonde évite un assèchement prématuré de celui-ci.

Lorsque la sonde 1 doit être utilisée, l'emballage 13 est descellé. Si la sonde 1 a été glissée dans la gaine 15, elle en est extraite et, peut être utilisée. Dans le cas où elle a été pré imprégnée du liquide de mouillage, l'utilisateur peut immédiatement l'introduire dans le conduit corporel correspondant.

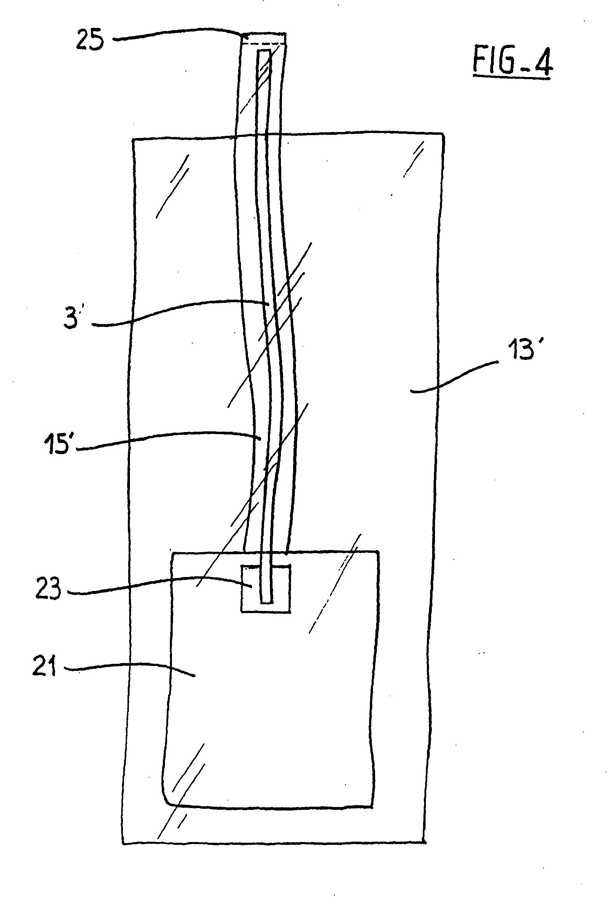

Sur la figure 4, le cathéter sonde 3' est un tube flexible biocompatible qui peut être fabriqué comme le cathéter 3, c'est-à-dire pour être au moins extérieurement hydrophile et mouillable par un agent de mouillage, avant utilisation.

Le cathéter 3' est fixé à une extrémité à une poche souple de récupération/stockage 21 avec laquelle elle est en communication en fluide 23.

Le cathéter 3' est placé, pour un conditionnement stérile, dans une gaine souple 15' pouvant avoir les caractéristiques de la gaine 15.

La gaine 15' est allongée suivant l'axe du cathéter et est fixée à la poche 21.

La gaine 15' est fermée par une zone déchirable 25, à l'opposé de la poche 21.

Hormis à proximité immédiate de la zone 25, la gaine souple 15' est elle-même conditionnée dans une pochette extérieure 13' pouvant être structurellement identique à l'emballage 13.

Le cathéter 3' ainsi conditionné est stérilisable au gaz (parois des "poches de conditionnement" qui l'entourent alors perméables).

Dans ce qui précède, on n'a fait référence qu'aux sondes urinaires.

Eventuellement, la sonde 1 peut être utilisée comme sonde (ou cathéter) anal(e), spécialement si un glissement "serré" (peu de jeu) est à réaliser.

Parmi les autres applications on peut noter les trocarts, les sondes sub-pubiennes et les cathéters (notamment d'angioplastie) équipés d'un ballon gonflable dans un vaisseau. Dans ce dernier ce pourrait n'être que la membrane du ballon qui serait conçu comme dans l'invention. Seuls certains éléments, ou certaines parties, d'un dispositif médical intraluminal plus complet pourraient donc être conçu(e)s comme prévu dans l'invention.

## Revendications

1. Ensemble comprenant :
- un dispositif médical intraluminal adapté pour être introduit dans un conduit corporel, le dispositif médical présentant au moins localement une zone hydrophile (3, 3') comprenant une première substance hydrophile et présentant extérieurement un faible coefficient de frottement consécutivement à un traitement de ladite zone avec un agent de mouillage de ladite première substance hydrophile, préalablement à l'utilisation de la sonde, la zone hydrophile (3) comprend à sec une deuxième substance hydrophile constituant un agent de rétention de l'agent de mouillage au sein de la zone hydrophile, pour limiter l'évaporation à l'air de cet agent de mouillage hors du dispositif médical et/ou pour éviter audit agent de mouillage de couler, comme de l'eau, une fois la zone traitée, le dispositif consistant de préférence en une sonde,
- un emballage (13) dans lequel le dispositif est conditionné mouillé, imprégné par son agent de mouillage, préalablement ou lors de son conditionnement dans l'emballage,
- et après imprégnation, pendant sa durée ultérieure de maintien dans l'emballage, le dispositif médical demeure dans son emballage sans y tremper dans aucun liquide,
- dans l'emballage, le dispositif médical est recouvert d'un gel de mouillage, à l'endroit de sa zone hydrophile (3), ce gel constituant l'agent de rétention de l'agent de mouillage.

2. Ensemble selon la revendication 1, **caractérisé en ce que** la zone hydrophile (3) comprend :
- une première couche (9) comprenant une proportion nettement plus importante de première substance hydrophile que de deuxième substance hydrophile, et
- une deuxième couche (11), autour de la première, comprenant une proportion nettement plus importante de deuxième substance hydrophile que de première substance hydrophile.

3. Ensemble selon l'une quelconque des revendications précédentes, **caractérisé en ce que** l'agent de rétention de l'agent de mouillage est un lubrifiant hygroscopique qui limite les frottements.

4. Ensemble selon l'une quelconque des revendications précédentes, **caractérisé en ce que** l'agent de rétention de l'agent de mouillage comprend de la glycérine.

5. Ensemble selon l'une quelconque des revendications précédentes, **caractérisé en ce que** la première substance hydrophile est adaptée pour gonfler en présence de l'agent de mouillage, lequel consiste alors en un agent de gonflement.

6. Ensemble selon l'une quelconque des revendications précédentes, **caractérisé en ce que** la sonde comprend un substrat central (30) recouvert de ladite zone hydrophile.

7. Ensemble selon l'une quelconque des revendications précédentes, **caractérisé en ce que** la deuxième substance hydrophile présente une consistance gélatineuse.

8. Ensemble selon l'une quelconque des revendications précédentes, **caractérisé en ce que** la zone hydrophile comprend au moins 40 % de deuxième substance hydrophile et entre 59,5% et 10 % de composé aqueux, en particulier de l'eau, en tant qu'agent de mouillage.

9. Ensemble selon la revendication 4 ou la revendication 8, **caractérisé en ce qu'**il comprend entre 70 % et 90 % de glycérine en tant qu'agent de rétention de l'agent de mouillage.

10. Procédé de préparation d'un dispositif médical intraluminal, tel en particulier qu'une sonde, dans un emballage, dans lequel procédé:
- on obtient un dispositif médical présentant au moins localement une zone hydrophile (3) comprenant une première substance hydrophile et présentant extérieurement un faible coefficient de frottement consécutivement à un traitement de ladite zone avec un agent de mouillage de ladite première substance hydrophile, préalablement à l'utilisation du dispositif médical,
- on conditionne ce dispositif médical dans un emballage (13),
- avant ou lors de l'étape de conditionnement, on traite le dispositif médical avec l'agent de mouillage pour qu'il s'en imprègne,
- et on stérilise l'intérieur de l'emballage,
**caractérisé en ce que**
- on prépare le dispositif médical pour qu'à l'intérieur de l'emballage (13) la zone hydrophile (3) comprenne une deuxième substance hydrophile constituant un agent de rétention de l'agent de mouillage, pour limiter l'évaporation à l'air de cet agent de mouillage hors du dispositif médical, une fois la zone traitée,
- et l'étape de stérilisation comprend une stérilisation gazeuse, à travers au moins une paroi (19) de l'emballage qui est perméable au gaz.

11. Procédé selon la revendication 10, **caractérisé en ce que** l'étape de préparation du dispositif médical comprend l'enduction d'une couche (11) comprenant la deuxième substance hydrophile autour d'une couche sous-jacente (9) comprenant la première substance hydrophile.

12. Procédé selon la revendication 10 au la revendication 11, **caractérisé en ce que** lors de l'étape de préparation du dispositif médical, on met en contact la première substance hydrophile avec à la fois la deuxième substance hydrophile et l'agent de mouillage.

13. Procédé selon l'une quelconque des revendications 10 à 12, **caractérisé en ce que,** lors de l'étape de préparation du dispositif médical, on enduit extérieurement sa zone hydrophile (3) avec un gel de mouillage (11) contenant une quantité de deuxième substance hydrophile et d'agent de mouillage.

## Claims

1. Assembly comprising:
- an intraluminal medical device which is suitable for being introduced into a bodily passage, the medical device having at least locally a hydrophilic zone (3, 3') which comprises a first hydrophilic substance and which has, at the outer side, a low friction coefficient following treatment of said zone with an agent for wetting the first hydrophilic substance before the probe is used, the hydrophilic zone (3) comprises, in the dry state, a second hydrophilic substance which constitutes an agent for retaining the wetting agent in the hydrophilic zone, in order to limit the evaporation of this wetting agent in air outside the medical device and/or to prevent said wetting agent from flowing in the manner of water after the zone has been treated, the device preferably comprising a probe,
- a packaging (13) in which the device is packaged in the wet state, impregnated with the wetting agent thereof, beforehand or when it is packaged in the packaging,
- and after impregnation, for the period of time for which it is subsequently retained in the packaging, the medical device remains in its packaging without being immersed there in any liquid,
- in the packaging, the medical device is covered with a wetting gel, at the location of the hydrophilic zone (3) thereof, this gel constituting the agent for retaining the wetting agent.

2. Assembly according to claim 1, **characterised in that** the hydrophilic zone (3) comprises:
- a first layer (9) comprising a significantly larger proportion of first hydrophilic substance than second hydrophilic substance, and
- a second layer (11), around the first, comprising a significantly larger proportion of second hydrophilic substance than first hydrophilic substance.

3. Assembly according to either of the preceding claims, **characterised in that** the agent for retaining the wetting agent is a hygroscopic lubricant which limits the occurrences of friction.

4. Assembly according to any one of the preceding claims, **characterised in that** the agent for retaining the wetting agent comprises glycerol.

5. Assembly according to any one of the preceding claims, **characterised in that** the first hydrophilic substance is suitable for swelling in the presence of the wetting agent which is composed of a swelling agent.

6. Assembly according to any one of the preceding claims, **characterised in that** the probe comprises a central substrate (30) covered by said hydrophilic zone.

7. Assembly according to any one of the preceding claims, **characterised in that** the second hydrophilic substance has a gel-like consistency.

8. Assembly according to any one of the preceding claims, **characterised in that** the hydrophilic zone comprises at least 40% of second hydrophilic substance and between 59.5% and 10% of aqueous compound, in particular water, as a wetting agent.

9. Assembly according to claim 4 or claim 8, **characterised in that** it comprises between 70% and 90% of glycerol as an agent for retaining the wetting agent.

10. Method for preparing an intraluminal medical device, such as in particular a probe, in a packaging, in which method:
- a medical device is obtained which has at least locally a hydrophilic zone (3) which comprises a first hydrophilic substance and which has, at the outer side, a low friction coefficient following treatment of said zone with a wetting agent of said first hydrophilic substance, before the medical device is used,
- this medical device is packaged in a packaging (13),
- before or during the packaging step, the medical device is treated with the wetting agent so that it becomes impregnated therewith,
- and the interior of the packaging is sterilised,
**characterised in that:**
- the medical device is prepared so that, at the inner side of the packaging (13), the hydrophilic zone (3) comprises a second hydrophilic substance which constitutes an agent for retaining the wetting agent in order to limit the evaporation of this wetting agent in air outside the medical device after the zone has been treated,
- and the sterilisation step comprises a gas-type sterilisation, through at least one wall (19) of the packaging which is gas-permeable.

11. Method according to claim 10, **characterised in that** the step for preparing the medical device comprises coating with a layer (11) which comprises the second hydrophilic substance around a subjacent layer (9) which comprises the first hydrophilic substance.

12. Method according to claim 10 or claim 11, **characterised in that**, during the step for preparing the medical device, the first hydrophilic substance is simultaneously placed in contact with the second hydrophilic substance and the wetting agent.

13. Method according to any one of claims 10 to 12, **characterised in that**, during the step for preparing the medical device, the hydrophilic zone (3) thereof is coated at the outer side with a wetting gel (11) which contains a quantity of second hydrophilic substance and wetting agent.

## Patentansprüche

1. Aufbau mit:
- einem intraluminalen medizinischen Gerät, das dazu geeignet ist, in einen Körpergang eingeführt zu werden, wobei das medizinische Gerät mindestens lokal eine hydrophile Zone (3, 3'), die eine erste hydrophile Substanz enthält, und außen einen geringen Reibungskoeffizienten nach einer Behandlung der Zone mit einem Benetzungsmittel der ersten hydrophilen Substanz vor der Verwendung der Sonde aufweist, die hydrophile Zone (3) trokken eine zweite hydrophile Substanz enthält, die ein Retentionsmittel des Benetzungsmittels im Inneren der hydrophilen Zone bildet, um die Verdunstung dieses Benetzungsmittels an der Luft außerhalb des medizinischen Gerätes zu begrenzen und/oder um zu vermeiden, daß das Benetzungsmittel wie Wasser fließt, wenn die Zone erst einmal behandelt wurde, wobei das Gerät vorzugsweise aus einer Sonde besteht,
- einer Umhüllung (13), in welcher das Gerät benetzt konditioniert ist, wobei es mit seinem Benetzungsmittel vor oder während seines Konditionierens in der Umhüllung getränkt wird,
- wobei das medizinische Gerät nach dem Tränken während seiner weiteren Verweildauer in der Umhüllung verbleibt, ohne dort in eine Flüssigkeit einzutauchen,
- das medizinische Gerät in der Umhüllung mit einem Benetzungsgel an der Stelle seiner hydrophilen Zone (3) bedeckt ist, wobei dieses Gel das Retentionsmittel des Benetzungsmittels bildet.

2. Aufbau nach Anspruch 1, **dadurch gekennzeichnet, daß** die hydrophile Zone (3) aufweist:
- eine erste Schicht (9) mit einem deutlich höheren Prozentsatz an erster hydrophiler Substanz als an zweiter hydrophiler Substanz, und
- eine zweite Schicht (11) um die erste herum, die einen deutlich höheren Prozentsatz der zweiten hydrophilen Substanz als der ersten hydrophilen Substanz enthält.

3. Aufbau nach einem der vorhergehenden Ansprüche**, dadurch gekennzeichnet, daß** das Retentionsmittel des Benetzungsmittels ein hygroskopisches Gleitmittel ist, welches die Reibungen begrenzt.

4. Aufbau nach einem der vorhergehenden Ansprüche, **dadurch gekennzeichnet, daß** das Retentionsmittel des Benetzungsmittels Glycerin enthält.

5. Aufbau nach einem der vorhergehenden Ansprüche, **dadurch gekennzeichnet, daß** die erste hydrophile Substanz geeignet ist, in Anwesenheit des Benetzungsmittels zu quellen, das also aus einem Quellmittel besteht.

6. Aufbau nach einem der vorhergehenden Ansprüche, **dadurch gekennzeichnet, daß** die Sonde ein zentrales Substrat (30) aufweist, das mit der hydrophilen Zone bedeckt ist.

7. Aufbau nach einem der vorhergehenden Ansprüche, **dadurch gekennzeichnet, dass** die zweite hydrophile Substanz eine gallertartige Konsistenz aufweist.

8. Aufbau nach einem der vorhergehenden Ansprüche, **dadurch gekennzeichnet, daß** die hydrophile Zone mindestens 40% an zweiter hydrophiler Substanz und zwischen 59,5% und 10% an wässriger Zusammensetzung, insbesondere Wasser, als Benetzungsmittel aufweist.

9. Aufbau nach Anspruch 4 oder nach Anspruch 8, **dadurch gekennzeichnet, daß** er zwischen 70% und 90% Glycerin als Retentionsmittel des Benetzungsmittels aufweist.

10. Herstellungsverfahren eines intraluminalen medizinischen Gerätes, wie z. B. insbesondere einer Sonde in einer Umhüllung, wobei man in diesem Verfahren:
- ein medizinisches Gerät erhält, das mindestens lokal eine hydrophile Zone (3) aufweist, die eine erste hydrophile Substanz enthält, und außen einen geringen Reibungskoeffizienten nach einer Behandlung der Zone mit einem Benetzungsmittel der ersten hydrophilen Substanz vor der Benutzung des medizinischen Gerätes aufweist,
- dieses medizinische Gerät in einer Umhüllung (13) konditioniert,
- vor oder während des Konditionierschrittes das medizinische Gerät mit dem Benetzungsmittel behandelt, damit es sich damit tränkt,
- und das Innere der Umhüllung sterilisiert,
**dadurch gekennzeichnet, daß:**
- man das medizinische Gerät herstellt, damit die hydrophile Zone (3) im Inneren der Umhüllung (13) eine zweite hydrophile Substanz aufweist, die ein Retentionsmittel des Benetzungsmittels bildet, um die Verdunstung dieses Benetzungsmittels an der Luft außerhalb des medizinischen Gerätes zu verhindern, wenn die Zone erst einmal behandelt wurde,
- und der Sterilisationsschritt eine gasförmige Sterilisation quer durch mindestens eine Wand (19) der Umhüllung, welche für Gas durchlässig ist, aufweist.

11. Verfahren nach Anspruch 10, **dadurch gekennzeichnet, daß** der Herstellungsschritt des medizinischen Gerätes den Überzug einer Schicht (11), welche die zweite hydrophile Substanz enthält, um eine darunter angrenzende Schicht (9) aufweist, welche die erste hydrophile Substanz enthält.

12. Verfahren nach Anspruch 10 oder nach Anspruch 11, **dadurch gekennzeichnet, daß** man während des Herstellungsschrittes des medizinischen Gerätes die erste hydrophile Substanz gleichzeitig mit der zweiten hydrophilen Substanz und dem Benetzungsmittel in Kontakt bringt.

13. Verfahren nach einem der Ansprüche 10 bis 12, **dadurch gekennzeichnet, daß** man während des Herstellungsschrittes des medizinischen Gerätes seine hydrophile Zone (3) außen mit einem Benetzungsgel (11) überzieht, welches eine Menge an zweiter hydrophiler Substanz und Benetzungsmittel enthält.
